Europäisches Patentamt

European Patent Office

Office européen des brevets

(19)

(11) **EP 0 602 230 B1**

(12) **EUROPÄISCHE PATENTSCHRIFT**

(45) Veröffentlichungstag und Bekanntmachung des Hinweises auf die Patenterteilung:
**08.01.1997 Patentblatt 1997/02**

(21) Anmeldenummer: **93915722.8**

(22) Anmeldetag: **28.06.1993**

(51) Int Cl.⁶: $G02B\ 5/28$, $A61F\ 9/02$

(86) Internationale Anmeldenummer:
**PCT/EP93/01647**

(87) Internationale Veröffentlichungsnummer:
**WO 94/01793 (20.01.1994 Gazette 1994/03)**

(54) **STRAHLENSCHUTZANORDNUNG MIT INTEGRIERTEM BESTRAHLUNGS-INDIKATOR**

RADIATION PROTECTION ARRANGEMENT WITH INTEGRATED EXPOSURE INDICATOR

SYSTEME DE PROTECTION CONTRE LES RAYONNEMENTS COMPORTANT UN INDICATEUR D'IRRADIATION

(84) Benannte Vertragsstaaten:
**AT BE CH DE ES FR GB IT LI NL**

(30) Priorität: **01.07.1992 DE 4221523**

(43) Veröffentlichungstag der Anmeldung:
**22.06.1994 Patentblatt 1994/25**

(73) Patentinhaber: **JENOPTIK Aktiengesellschaft 07739 Jena (DE)**

(72) Erfinder:
• **HACKER, Erik D-07747 Jena (DE)**
• **POHLACK, Huber D-07743 Jena (DE)**

(74) Vertreter: **Geyer, Werner, Dr.-Ing. et al Patentanwälte GEYER & FEHNERS Perhamerstrasse 31 80687 München (DE)**

(56) Entgegenhaltungen:
**EP-A- 0 080 182          WO-A-93/09449**
**FR-A- 2 479 482**

**Beschreibung**

Die Erfindung betrifft eine Anordnung zum Schutz gegen schädigende Einwirkungen von Strahlen und zu deren Indikation, die vorzugsweise als Sichtfenster oder Brille gestaltet zum Schutz gegen Schädigungen der Augen gegenüber elektromagnetischer Strahlung hoher Bestrahlungsstärke verwendet werden kann.

Strahlenschutzvorrichtungen, insbesondere zum Schutz der Augen gegenüber energiereicher monochromatischer Strahlung hoher Flächendichte, zum Beispiel Laserstrahlung, sind in zahlreichen Ausführungsformen bekannt. Sie sind meist als Platten bzw. Folien aus transparentem Material ausgebildet und mit Überfangmaterialien versehen, welche eine solche Spektralfilterwirkung aufweisen, daß der Spektralbereich der Strahlung, vor welchem die Augen geschützt werden sollen, am Durchdringen weitgehend gehindert, der nicht gefährdende Spektralbereich hingegen in einem solchen Maße hindurchgelassen wird, daß die Beobachtung der Vorgänge in dem strahlungsgefährdeten Raum, insbesondere in unmittelbarer Umgebung der Strahlungsquelle, möglich ist.

Derartige Überfangmaterialien auf transparenten Trägern sind häufig als Folgen dünner Interferenzschichten ausgeführt, deren Substanzeigenschaften und Schichtdicken in bekannter Weise so ausgewählt sind, daß das Interferenzschichtsystem im Spektralbereich der gefährdenden Strahlung einen hohen Reflexionsgrad (nahe 100%), in den benachbarten Spektralbereichen jedoch eine nur relativ geringe Reflexion aufweist.

So sind z.B. aus der Literatur: "Infrared Filters" von A. Borisevich, V.G. Vereshehagain, und M.A.Validov, 1971, S. 206 - 208 Konstruktionsprinzipien der Lichtfilterung durch das Zusammenwirken der Spektralfunktionen von Interferenzfiltern (z.B. Schmalbandfilter vom Fabry-Perot-Typ oder Multi-Cavity-Filter) und Absorptionsfiltern bekannt. Die hier beschriebenen Konstruktionsprinzipien bewirken die Blockung der aufgrund der Periodizität bei Interferenzfiltern auftretenden Transmissionsbereiche höherer Ordnung durch Kombination von Interferenzfiltern mit absorbierenden Substraten und/ oder Superstraten (z.B. Kittgruppen), die eine Kurzund/oder Langpasscharakteristik aufweisen. Die Ergänzung dieser Kombination mit zusätzlichen meist absorbierenden Interferenzschichtfiltern als Blockgruppen (Kurzpässe, Langpässe) führen zur Verbesserung der Lichtblockfunktion. So z.B. blocken die im IR-Bereich häufig verwendeten Ge/ZnS-Langpassblockfilter aufgrund der Absorption des Germaniums den gesamten sichtbaren Spektralbereich.

Aus H. A. M. Macleod "Thin-Film Optical Filters" Adam Hilger Ltd., Bristol (1986), S. 292 - 308 sind Metallinterferenzfilter vom Typ Fabry-Perot bekannt, bei denen die schmalbandigeTransmissionscharakteristik durch eine beidseitige Einbettung einer dielektrischen Abstandsschicht in halbdurchlässige Metallschichtspiegel erzeugt wird.

Desweiteren sind aus B.V. Landan, P.H. Lissberger "Theory of Induced Tranmission Filters in Terms of Concept of Equivalent Layers", JOSA 62 (1972) No. 11, P. 1258 - 1264 Schichtsysteme bestehend aus absorbierenden und nichtabsorbierenden Schichten mit induzierter Transmission bekannt, bei denen die Absorptionsproblematik durch geeignete Beeinflussung der Feldstärken des stehenden elektrischen Feldes in den Orten der absorbierenden Schichten gemindert wird.

Die Verwendung der beschriebenen Schichtsysteme für Strahlenschutzanordnungen bewirkt, daß die schädigende Strahlung von den Augen des im Bereich des Strahlungsfeldes arbeitenden Operators bzw. weiterer, in unmittelbarer Umgebung anwesender Personen zuverlässig ferngehalten wird. Andererseits weisen derartige bekannte Anordnungen insofern schwerwiegende Nachteile auf, als der Benutzer einer solchen Schutzvorrichtung grundsätzlich nicht feststellen kann, ob bzw. wann er einer Strahlungsgefährdung ausgesetzt ist und auch im Anschluß an den Aufenthalt in der strahlungsgefahrdenden Zone eine eingetretene Strahlungsexposition nicht mehr nachweisbar ist, so daß Strahlungsgefährdungen nicht erkannt und ihre Ursachen daher auch nicht beseitigt werden können.

Der Erfindung liegt die Aufgabe zugrunde, eine gegen die gefährdende Strahlung einer Wellenlänge $\lambda_0$ wirksame Strahlenschutzvorrichtung mit selbsttätiger Indikatorwirkung zu schaffen, bei welcher beim Auftreffen der gefährdenden Strahlung eine in Durchsicht und/oder Reflexion insbesondere visuell nachweisbare lokale Veränderung von Teilen des Materials der Schutzvorrichtung erzeugt wird. Dabei soll die Schutzwirkung der Strahlenschutzanordnung zu keinem Zeitpunkt vor, während oder nach einer irreversiblen oder reversiblen Veränderung beeinträchtigt sein.

Diese Aufgabe wird mit einer erfindungsgemäßen Anordnung gemäß Anspruch 1 gelöst.

Die erfindungsgemäße Anordnung besteht aus einer transparenten Trägerplatte,-scheibe oder -folie und einer auf einem solchen Träger aufgebrachten Folge dünner optischer Schichten, welche hinsichtlich Schichtmaterialien und Schichtdicken so ausgewählt und angeordnet sind, daß ein hoher Schutz gegen Transmission des gefährdenden Spektralintervalls bei ausreichender Durchsicht im übrigen sichtbaren Spektralbereich gewährleistet und mit der gleichen Anordnung die Kontrolle über Strahlungsexpositionen ermöglicht wird.

Die Selektierung der gefährdenden Strahlung der Wellenlänge $\lambda_0$ und die Verhinderung ihrer Transmission erfolgt zunächst nicht, entsprechend den bekannten Dünnschicht-Strahlenschutzvorrichtungen, vorzugsweise durch Reflexion, sondern vorzugsweise durch selektive Absorption innerhalb einer Schicht des Schutz-Schichtsystems. Die stattfindende Wandlung von Strahlungsenergie in Wärmeenergie erzeugt in der absorbierenden Schicht der erfindungsgemäßen Vorrichtung bei erhöhter Temperatur, je nach den physikalischen und chemischen Schichtsubstanzeigenschaften, unterschiedliche strukturelle und/oder chemische Veränderungen, zum Beispiel lokale Schichtabtragungen (Ablation), Materialaufwerfungen, Bläschenbildung, Texturveränderungen, Verfärbungen, Oxidation oder andere Phasen- und Modifikationsänderungen, welche nach einem Grundgedanken der Erfindung als sichtbare Materialinhomogenitäten zur Indikation einer stattgefundenen Strahlungsexposition der Schutzvorrichtung dienen. Diese Materialinhomogenitäten können irreversibel oder reversibel sein. Führt mindestens einer der genannten Degradationsprozesse zur Aufhebung der selektiven Absorption in der absorbierenden Schicht des Schichtsystems, so sorgt das nachgelagerte Substrat-Schichtsystem für die Aufrechterhaltung des Strahlenschutzes vorzugsweise durch Reflexion oder durch eine kombinierte Wirkung von Reflexion und Absorption.

Die Indikations- oder Konversions-Empfindlichkeit einer dünnen absorbierenden Schicht zur Erzeugung sichtbarer Materialveränderungen, die auf fotothermischen oder fotochemischen Energiewandlungseffekten beruhen, ist bekanntlich definiert als:

$$R_c = const \cdot (1/Q_{rad,\,min}),$$

wobei $Q_{rad,\,min}$ die auftreffende Strahlungsenergie bei der Wellenlänge $\lambda_0$ bedeutet, die mindestens erforderlich ist, um den Wandlungseffekt auszulösen. Bei konstantem Strahlquerschnitt wird der Schwellenwert $Q_{rad,\,min}$ um so kleiner und damit die Indikationsempfindlichkeit um so größer, je kleiner die Dichte $\rho$ sowie die spezifische Wärme c des absorbierenden Schichtmaterials und je geringer die für die Materialveränderung erforderliche Temperaturerhöhung (zum Beispiel bis zur Schmelztemperatur) sind und je größer die Absorptionsdichte A/d ist mit A als der in der Schicht der Dicke d absorbierte Anteil der auf die Schutzanordnung auftreffenden Strahlungsenergie bei der Wellenlänge $\lambda_0$.

Die für die Indikations-Empfindlichkeit relevante Größe A/d erfährt in der erfindungsgemäßen Anordnung eine entscheidende Steigerung gegenüber konventionellen optischen Absorptionsprozessen dadurch, daß die gesamte Schichtenfolge als optisches Resonanzabsorbersystem nach POHLACK ausgebildet ist (DD-WP 146 224, DD-WP 152 638), in welchem die absorbierende Schicht die Funktion des absorbierenden Resonators für die Wellenlänge $\lambda_0$ der gefährdenden Strahlung einnimmt. Ein solches Schichtsystem erzeugt bekanntlich - im Gegensatz zu der exponentiell abklingenden Absorption in nicht resonanzfähigen, ausgedehnten Absorbern des gleichen Materials - eine hundertprozentige oder nahezu hundertprozentige Absorption bereits innerhalb Schichten mit einer extrem geringen Dicke, erreicht also extrem hohe Absorptionsdichten A/d im Spektralbereich um die Wellenlänge $\lambda_0$ und trägt damit

zu der erwünschten Absenkung der Energieschwelle für das Auslösen signifikanter fotothermischer (oder fotochemischer) Wandlungseffekte bei.

Um den schichttechnologischen Aufwand für den Strahlenschutz und die Strahlungs-Indikation gering zu halten, soll gemäß der Erfindung das für die absorbierende Resonanzschicht auszuwählende Material in dem Spektralbereich, der die Wellenlänge $\lambda_0$ der gefährdenden Strahlung enthält, einen Absorptionskoeffizienten k größer als 0.01 und vorzugsweise größer als 1 aufweisen.

Darüber hinaus ist es, wie die obigen Ausführungen über den fotothermischen Wandlungsprozeß aufzeigen, zur Erzielung einer möglichst empfindlichen Strahlungs-Indikation zweckmäßig, aus den für den gewünschten fotothermischen Effekt geeigneten absorbierenden Materialien diejenigen auszuwählen, welche eine relativ geringe spezifische Wärme und eine relativ geringe Massendichte aufweisen.

Gemäß der Erfindung ist der die rückseitige Resonatorwand repräsentierende, in Strahlungsrichtung hinter der Resonanzschicht angeordnete Teil des gesamten Schichtsystems ausgebildet als eine mindestens im Spektralbereich der gefährdenden Strahlung einen hohen Reflexionsgrad aufweisende Kombination bestehend aus brechzahldifferenten Interferenzschichten, die nicht oder nur sehr gering absorbieren, mit einer auf die Strahlungsrichtung bezogenen abwechselnden Folge niedriger und hoher bzw. hoher und niedriger Schichtbrechzahlen. Zur Erzielung einer hohen Resonatorgüte soll die auf die der Resonanzschicht anliegende Grenzfläche des hinteren Interferenzschichtsystems bezogene Pseudobrechzahl, welche in bekannter Weise die optischen Eigenschaften des nachfolgenden Teilschichtsystems integriert, bei der Wellenlänge $\lambda_0$ Werte gleich oder kleiner als 0.1 aufweisen, wenn die Wechselschichtfolge in Strahlungsrichtung mit der kleineren Schichtbrechzahl beginnt bzw. Werte gleich oder größer als 10, wenn die Wechselschichtfolge mit der größeren Schichtbrechzahl beginnt.

Die genannten Pseudobrechzahlen werden erzielt, wenn das der Resonatorschicht nachgeordnete Teilschichtsystem in einem die Wellenlänge $\lambda_0$ enthaltenden Spektralintervall innerhalb des Wellenlängenbereichs $\lambda_0$ - 50 nm und $\lambda_0$ + 50 nm einen Reflexionsgrad gegen Luft aufweist, der größer als 99% ist. Dabei dient diese hohe Reflexion bei der Wellenlänge $\lambda_0$ zugleich dem Strahlenschutz, wenn die absorbierende Schicht durch den fotothermischen Wandlungsprozeß strahlungsdurchlässig oder teilweise strahlungsdurchlässig geworden ist. Zur Erzielung hinreichender Durchsichtigkeit der Schutzanordnung, also hinreichender Lichtdurchlässigkeit mindestens in Teilen des sichtbaren Spektrums soll der Reflexionsgrad der hinter der absorbierenden Resonatorschicht angeordneten Schichtkombination im sichtbaren Spektralbereich unter Ausschluß des die Wellenlänge $\lambda_0$ enthaltenden Spektralintervalls kleiner als 50% sein.

Gemäß der Erfindung ist der die vordere Resonatorwand repräsentierende, in Strahlungsrichtung vor der Resonanzschicht angeordnete Teil des gesamten Schichtsystems als reflexionsauslöschende Schicht oder Schichtkombination ausgebildet, welche die reflexionsfreie Anpassung an das Superstrat, zum Beispiel Luft, bewirkt. In Fällen, in denen eine optische Anpassung an das Superstrat durch Interferenzschichten nicht erforderlich ist, zum Beispiel, wenn das Superstrat bereits die optische Funktion der vorderen Resonatorwand erfüllt oder wenn die Resonanzbedingung zwar nicht vollkommen, aber bereits hinreichend gut erfüllt wird, sieht die erfindungsgemäße Anordnung den unmittelbaren Kontakt der Resonanzschicht mit dem Superstrat oder das Zwischenschalten einer bei der Wellenlänge $\lambda_0$ optisch unwirksamen Schicht für den mechanischen und/oder Korrosionsschutz vor, wobei eine solche Schicht mit der Brechzahl $n_s$ die geometrische Dicke

$$d_s = m \cdot \lambda_0 / (2 \cdot n_s)$$

aufweist mit m als eine positive ganze Zahl.

Aufgrund der angegebenen Merkmale grenzt sich die erfindungsgemäße Schichtanordnung von solchen Materialanordnungen ab, die zum Zwecke der optischen Datenaufzeichnung zwar ebenfalls fotothermische, mittels Laserstrahlung erzeugte Wandlungsprozesse in absorbierenden Materialien ausnutzen, jedoch prinzipiell abweichende Schichtfolgen vorsehen, welche nicht den optischen Schicht-Resonanzbedingungen genügen oder welche, im Falle resonanzabsorptionsähnlicher Anordnungen, rückseitige Resonatorwände aus Metall oder Metall und dünne Zwischenschichten mit den bekannten erheblichen thermischen Verlusten einsetzen, so daß bei keiner dieser Anordnungen die Voraussetzungen für die hohe Indikations-Empfindlichkeit der fotothermischen Wandlung gegeben sind. Durch die Metallschicht sind derartige Anordnungen undurchsichtig und sind daher grundsätzlich für eine Anwendung einer im sichtbaren Spektralbereich durchsichtigen Strahlenschutzanordnung nicht geeignet.

In Fällen, in denen trotz der vorgeschlagenen Steigerung der fotothermischen Wandlungseffektivität mit Hilfe der Steigerung der Absorptionsdichte A/d die Schwellenwerte für das Auslösen eines fotothermischen (oder fotochemischen) Effektes innerhalb der absorbierenden Schicht aufgrund zu geringer Bestrahlungsstärken noch nicht erreicht werden, wird nach einem weiteren Grundgedanken der Erfindung mittels Modifizierung der Anordnung die Indikations-Empfindlichkeit zusätzlich gesteigert und auf diese Weise der Nachweis einer solchen energieärmeren Strahlung ermöglicht.

Dazu enthält eine erfindungsgemäße Anordnung eine zusätzliche Schicht, deren Material bei Temperaturen, die in der absorbierenden Schicht noch keine sichtbaren fotothermischen Wandlungseffekte hervorbringen, bereits deutliche Materialveränderungen aufweist, zum Beispiel lokales Schmelzen, Sublimieren, Verdampfen, Verfärbungen oder andere temperaturbedingte Erscheinungen. Diese Zusatzschicht, die vorzugsweise im unmittelbaren Kontakt mit der Absorptionsschicht angebracht ist, kann im Spektralbereich mit der gefährdenden Strahlung der Wellenlänge $\lambda_0$ absorptionsfrei sein, so daß die in dieser Zusatzschicht infolge Temperaturerhöhung auftretenden Prozesse nicht durch fotothermische Wandlung, sondern vorzugsweise durch den Wärmekontakt mit der Absorptionsschicht, also durch Wärmeleitung hervorgerufen werden. Dabei bezieht eine erfindungsgemäße Anordnung auch solche Zusatzschichten ein, die außerhalb eines die Wellenlänge $\lambda_0$ enthaltenden Spektralintervalls absorbierende Spektralbereiche aufweisen, die zum Beispiel durch Einfärben des Zusatzschichtmaterials (beispielsweise als Dye-in-Polymer) erzeugt werden. Eine solche Maßnahme erweist sich im Sinne des Erfindungsgedankens zum Beispiel dann als zweckmäßig, wenn die Wellenlänge $\lambda_0$ außerhalb des sichtbaren Spektrums liegt und eine deutliche Anzeige der Strahlungsexposition im Sichtbaren erreicht werden soll.

Ausführungsbeispiele

Die Erfindung wird anhand von Ausführungsbeispielen näher erläutert.

Dazu zeigen:

Fig. 1　den Verlauf des Reflexionsgrades R und
Fig.2　den spektralen Verlauf des Transmissionsgrades T des die rückseitige Resonatorwand repräsentierenden Schichtsystems gemäß dem ersten Ausführungsbeispiel

weiterhin zeigen:

Fig.3　den spektralen Verlauf des Reflexionsgrades R
Fig.4 und Fig.5　den spektralen Verlauf des Transmissionsgrades T den spektralen Verlauf des Absorptionsgrades A eines Dünnschicht-Resonanzabsorber-Systems gemäß dem ersten Ausführungsbeispiel.

In den Ausführungsbeispielen 1 bis 7 werden absorbierende optische Medien als Resonanzschicht eingesetzt, bei denen die bekannten Resonanzabsorptions-Bedingungen sehr geringe Resonanzschichtdicken d zulassen, so daß sehr hohe Absorptionsdichten A/d erreicht werden. Die betreffenden Schichtmaterialien, zu denen einige Metalle, Halbmetalle und deren Legierungen, die metallähnlichen Chalkogene sowie eine Reihe von Chalkogeniden zählen, grenzen sich von anderen chemischen Elementen und Verbindungen dadurch ab, daß die reelle Brechzahl n und der Absorptionskoeffizient k, also Realteil und Imaginärteil der komplexen Brechzahl n - ik, größer als 1 sind.

In weiteren, hier nicht weiter behandelten Ausführungsbeispielen, können absorbierende optische Medien als Resonanzschicht eingesetzt werden, bei denen aufgrund der Resonanzabsorptions-Bedingungen die Resonanzschichtdicken größer als bei den oben beschriebenen optischen Medien sein müssen. Die betreffenden Schichtmaterialien sind durch Absorptionskoeffizienten k gekennzeichnet, die kleiner als 1, vorzugsweise klein gegen 1 sind. Trotz der im Vergleich zur ersten Materialgruppe erzielbaren geringeren Absorptionsdichten A/d haben auch diese Materialien praktische Bedeutung für die erfindungsgemäßen Strahlenschutz- und Strahlenindikations-Anordnungen, weil sie in einigen Fällen eine hohe materialbedingte Temperaturempfindlichkeit aufweisen.

1. Ausführungsbeispiel

Die Schichtanordnung dieses Ausführungsbeispiels ist auf den Schutz gegen Laserstrahlung der Wellenlänge $\lambda_0$ = 633 nm und auf deren Indikation ausgelegt. Die absorbierende Resonatorschicht besteht aus Chrom mit der im mittleren sichtbaren Spektralbereich gemessenen komplexen Brechzahl n - ik $\approx$ 2.49 - i2.3. Diese Schichtsubstanz gehört also zu der Gruppe der Materialien mit n $\approx$ k und n > 1, k > 1, für welche die bekannten Resonanzbedingungen N (PB/Substrat) $\approx$ 0 und

$$N \text{ (PB/Resonator )} \approx 4 \cdot \pi \cdot n \cdot k / \lambda_0 \cdot d$$

gelten, worin N (PB/Substrat) bekanntlich die Pseudobrechzahl des die rückseitige Resonatorwand repräsentierenden, hinter der Resonatorschicht angeordneten Interferenzschichtsystems und N (PB/Resonator) die Pseudobrechzahl der Vorderfläche der absorbierenden Resonatorschicht ist; beide Pseudobrechzahlen sind bezogen auf die Wellenlänge $\lambda_0$.

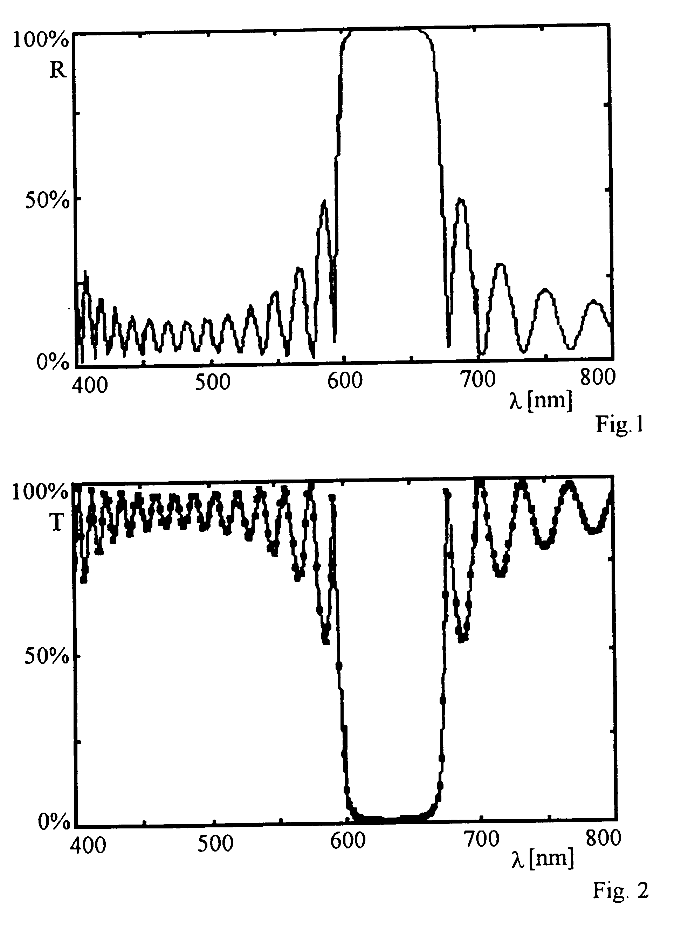

Die Pseudobrechzahl N (PB/Substrat), die gemäß den bekannten Resonanzbedingungen bei den mäßig bis hoch absorbierende Materialien (positive) Werte << 1 aufweisen muß, wird durch ein hinter der Resonatorschicht angeordnetes aus 16 Schichten abwechselnd aus Magnesiumfluorid und Siliziumnitrid bestehendes Wechselschichtsystem eingestellt. Die Schichtdicken betragen dabei jeweils 3 $\lambda_0$ /4, womit N (PB/Substrat) $\approx$ 0,0027 wird, also, wie gefordert, einen Wert < 0,1 erreicht. Das der absorbierenden Resonanzschicht nachgelagerte Interferenzschichtsystem kann vorteilhafterweise auch rein oxidisch, beispielsweise aus $SiO_2$ als niedrigbrechende Substanz und $TiO_2$, $ZrO_2$, $Ta_2O_5$ usw. als hochbrechende Substanz, aufgebaut sein. Reflexionsgrad R und Transmissionsgrad T der auf Glas aufgebrachten Interferenzschichtkombination des Ausführungsbeispiels weisen, wie die Fig. 1 und 2 zeigen, bei der Resonanzwellenlänge eine sehr hohe Reflexion, also eine sehr niedrige Transmission und im sichtbaren Spektralbereich außerhalb des die Resonanzwellenlänge enthaltenden schmalen Spektralintervalls eine nur mäßige Reflexion und damit eine ausreichende Transmission im übrigen sichtbaren Spektrum auf. Bei einer Schichtdicke der Resonatorschicht von $\approx$ 9 nm verschwindet die Reflexion gegen Luft für das Dünnschicht-Resonanzabsorber-System. Der Resonanz-Absorptionseffekt mit R $\approx$ 0, T $\approx$ 0, A $\approx$ 100 % und mit der für den fotothermischen oder fotochemischen Energiewandlungseffekt relevanten Absorptionsdichte A/d $\approx$ 1/9 nm$^{-1}$ > 0.1 nm$^{-1}$ bei der Resonanzwellenlänge $\lambda_r$ = $\lambda_0$ wird bereits ohne zusätzliche, vor der Resonatorschicht angebrachte Schichten erreicht:

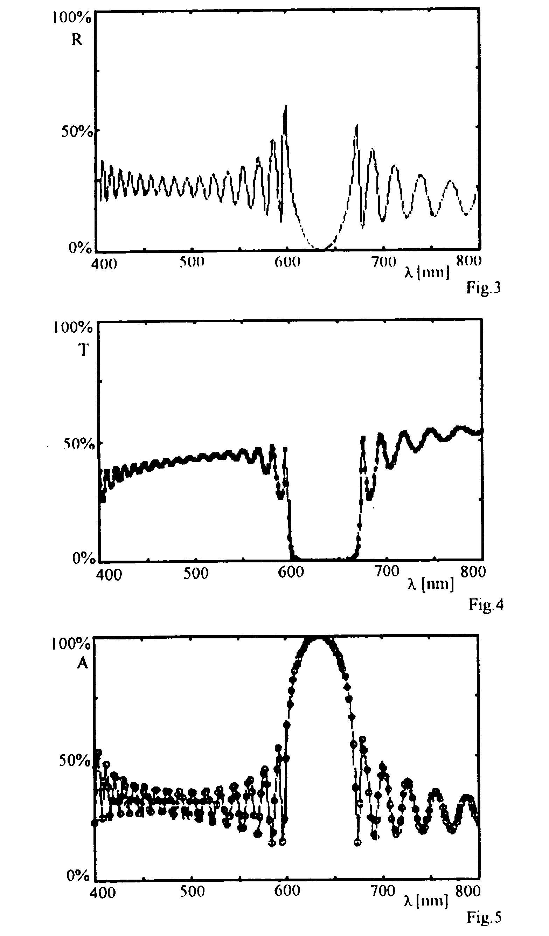

Die Fig. 3 bis 5 zeigen die spektralen Verläufe von Reflexionsgrad R, Transmissionsgrad T und Absorptionsgrad A des Dünnschicht - Resonanzabsorber-Systems.

Die fotothermische Wandlung bewirkt bei hinreichender Bestrahlungsstärke lokale Schichtzerstörungen durch lokales Schmelzen des Metalls, bei hohen Bestrahlungsstärken können substanzfreie Bereiche in der Resonanzschicht (Löcher) entstehen.

Die reflexionsfreie Anpassung ohne Zusatzschichten durch die Wahl der entsprechenden Resonanzschichtdicke bietet Vorteile für die Beschichtungstechnologie insofern, als die Aufbringung der Resonatorschicht mit optimaler Schichtdicke über das Reflexionsminimum (gegen Vakuum) erfolgen kann.

2. Ausführungsbeispiel

Dieses Beispiel unterscheidet sich vom 1. Ausführungsbeispiel durch die mit d $\approx$ 3 nm extrem reduzierte Resonanzschichtdicke. Bei gleichem Interferenzschichtsystem hinter der Resonatorschicht, also gleicher Pseudobrechzahl N (PB/Substrat) wie im 1. Ausführungsbeispiel, wird N (PB/Resonator) $\approx$ 0.34, ein Wert, der durch eine Zweifachschicht, bestehend aus Magnesiumfluorid und Titanoxid, jeweils mit der optischen Schichtdicke $\lambda_0$ /4, an das Superstrat Luft reflexionsfrei angepaßt ist.

Bei hinreichender Bestrahlungsstärke erzeugt die fotothermische Wandlung, trotz der Bedeckung der Resonatorschicht durch die vorderen Anpassungsschichten, sichtbare Materialinhomogenitäten aufgrund lokaler Schmelzprozesse.

3. Ausführungsbeispiel

Bei diesem Ausführungsbeispiel wird Tellur als Material für die Resonanzschicht eingesetzt. Tellur gehört ebenfalls zu der Gruppe der mäßig bis stark absorbierenden Resonanzschicht-Materialien mit n $\approx$ k (komplexe Brechzahl im mittleren sichtbaren Spektralbereich: n - ik $\approx$ 4.5 - i3), ist aber aufgrund des niedrigen Schmelzpunktes temperaturempfindlicher als Chrom, allerdings auch empfindlicher gegen Umwelteinflüsse. Im Ausführungsbeispiel ist die Resonanzschichtdicke mit d ≈ 6.5 nm angesetzt, so daß die Anpassung an das Superstrat Luft bei der Wellenlänge $\lambda_0 = 633$ nm gewährleistet ist. Zum Schutz gegen Zerstörung der Tellurschicht durch atmosphärische Einflüsse, insbesondere gegen Luftfeuchtigkeit, ist zwischen die Tellur-Resonatorschicht und das Superstrat Luft eine Schutzschicht aus Siliziumdioxid mit der optischen Schichtdicke $\lambda_0/2$ angeordnet, die bei der Wellenlänge $\lambda_0$ bekanntlich keine optische Wirkung zeigt.

### 4. Ausführungsbeispiel

In diesem Ausführungsbeispiel wird für die Wellenlänge $\lambda_0 = 633$ nm Tellur als Resonanzschicht mit einer Dicke von d ≈ 3 nm eingesetzt. Bei einer Gestaltung des in Strahlenrichtung gesehen nachgelagerten Interferenzschichtsystems analog Ausführungsbeispiel 1 gelingt die reflexionsfreie Anpassung durch eine aus einer hochund einer niedrigbrechenden Substanz aufgebauten Zweifachschicht, beispielsweise bestehend aus Magnesiumfluorid und Siliziumnitrid oder Siliziumoxid und Zirkoniumdioxid oder ähnliche Kombinationen.

### 5. Ausführungsbeispiel

In diesem Ausführungsbeispiel wird für die Wellenlänge $\lambda_0 = 488$ nm Wismut als Resonanzschicht mit einer Dicke von d ≈ 9 nm eingesetzt. Die komplexe Brechzahl beträgt bei dieser Wellenlänge n - ik ≈ 1.6 - i2.7. Bei einer Gestaltung des in Strahlenrichtung gesehen nachgelagerten Interferenzschichtsystems analog Ausführungsbeispiel 1 gelingt die reflexionsfreie Anpassung beispielsweise mit Siliziumnitrid oder einer Substanz mit einer ähnlichen Brechzahl, wobei die exakte reflexionsfreie Anpassung durch Dickenmodifizierung erreicht werden kann. Diese vorgelagerte Schicht erfüllt zudem eine Schutzfunktion.

### 6. Ausführungsbeispiel

Dieses Beispiel unterscheidet sich vom 5. Ausführungsbeispiel durch die auf d ≈ 4 nm reduzierte Dicke der Wismutschicht. Bei einem Interferenzschichtsystem hinter der Resonatorschicht analog Ausführungsbeispiel 1, in dem jedoch $\lambda_0/4$ - anstelle von $3\lambda_0/4$-Schichten gewählt wurden, gelingt eine reflexionsfreie Anpassung durch ein Zweifachschichtsystem, beispielsweise aus Magnesiumfluorid und Siliziumnitrid oder Substanzkombinationen mit ähnlichen Brechzahlen.

### 7. Ausführungsbeispiel

In diesem Ausführungsbeispiel wird für eine Wellenlänge $\lambda_0 \approx 800$ nm Antimontellurid als Resonanzschicht gewählt, das aufgrund eines reversiblen fotothermischen Wandlungsprozesses zur reversiblen Indikation von schädigender Strahlung geeignet ist. Bei einer Wahl des nachgelagerten Interferenzschichtsystems wie im 1. Ausführungsbeispiel kann mit einer Schutzschicht (z.B. Siliziumdioxid) bei einer geringfügigen Dickenmodifizierung eine exakte reflexionsfreie Anpassung der kristallinen Ausgangsmodifikation des Antimontellurids an das Superstrat Luft erreicht werden.

Die Ausführungsbeispiele 1 bis 7 erläutern Anordnungen, welche durch eine temperaturhochempfindliche Zusatzschicht erweiterbar sind, die sich in unmittelbarem oder vermitteltem Kontakt zur absorbierenden Resonanzschicht befinden. Solche Zusatzschichten können niedrigschmelzende Metalle, Halbleiter oder Dielektrika sein, die bei niedrigen Temperaturen reversible oder irreversible Veränderungen zeigen. In besonders vorteilhaften Ausführungsformen sind diese Zusatzschichten Schichten aus Polymeren oder Dye-in-Polymeren, die mittels bekannter Technologien wie spinning oder dipping aufgebracht werden.

### Patentansprüche

1. Anordnung zum Schutz gegen unter beliebigen Einfallswinkeln auftreffende elektromagnetische Strahlung mindestens einer Wellenlänge $\lambda_0$, bestehend aus einer mindestens im sichtbaren Spektralbereich transparenten Unterlage und mehreren brechzahldifferenten Interferenzschichten, gekennzeichnet dadurch,

   daß zusätzlich eine absorbierende Resonatorschicht vorhanden ist, welche gemeinsam mit den brechzahldifferenten Interferenzschichten eine Folge von optischen Schichten bildet, die nach dem Prinzip eines optischen Dünnschicht-Resonanzabsorbersystems mit mindestens einer Resonanzwellenlänge $\lambda_r = \lambda_0$ ausgewählt und angeordnet sind, wobei die in Strahlungsrichtung der absorbierenden Resonatorschicht nachgeordneten Interferenzschichten mindestens im Spektralbereich zwischen 450 nm und 750 nm absorptionsfrei sind und
   daß die absorbierende Resonatorschicht eine bestimmte Indikationsempfindlichkeit $R_c$ aufweist, so daß bei Überschreitung der auftreffenden Strahlungsenergie bei der Wellenlänge $\lambda$ eines Schwellwertes $Q_{rad, min}$ nachweisbare Materialveränderungen insbesondere durch fotochemische oder fotothermische Energiewandlungseffekte auftreten.

2. Anordnung nach Anspruch 1, gekennzeichnet dadurch,
   daß die in Strahlungsrichtung der absorbierenden Resonatorschicht nachgeordneten Interferenzschichten aus Materialien mit abwechselnd niedrigen und hohen oder abwechselnd hohen und niedrigen Brechzahlen besteht.

3. Anordnung nach Anspruch 2, gekennzeichnet dadurch,

daß die optischen Dicken $n_s \cdot d_s$ der in Strahlungsrichtung der absorbierenden Resonatorschicht nachgeordneten Interferenzschichten mit $n_s \cdot d_s = (m+1) \lambda_0/4$ bemessen sind, wobei $n_s$ die jeweilige Brechzahl der Einzelschicht, $d_s$ die jeweilige geometrische Dicke der Einzelschicht und m gleich Null oder eine natürliche Zahl ist und

daß die in Strahlungsrichtung der absorbierenden Resonatorschicht nachgeordneten Interferenzschichten in einem die Wellenlänge $\lambda_0$ enthaltenden Spektralintervall innerhalb des Wellenlängenbereiches von $0{,}9 \cdot \lambda_0$ bis $1{,}1 \lambda_0$ einen Reflexionsgrad gegen Luft größer als 99% und im sichtbaren Spektralbereich außerhalb des Wellenlängenbereiches von $0{,}9 \lambda_0$ bis $1{,}1 \lambda_0$ einen Reflexionsgrad gegen Luft kleiner als 50% aufweisen, wodurch die in Strahlungsrichtung hinter der absorbierenden Resonanzschicht angeordnete Teilfolge von optischen Schichten in ihrer Grenzfläche zur Resonanzschicht bei der Wellenlänge $\lambda$ eine Pseudobrechzahl aufweist, welche $\leq 0{,}1$ ist, wenn die Teilfolge mit einer Schicht der kleinen Brechzahl beginnt und welche $\geq 10$ ist, wenn die Teilfolge mit einer Schicht der größeren Brechzahl beginnt.

4. Anordnung nach Anspruch 1, gekennzeichnet dadurch,
daß das Material der absorbierenden Resonatorschicht mindestens bei einer Wellenlänge $\lambda_0$ einen Absorptionskoeffizienten k größer als 1 aufweist.

5. Anordnung nach Anspruch 4, gekennzeichnet dadurch,
daß das Material der absorbierenden Resonatorschicht eine Dichte $\rho$, und eine spezifische Wärme c aufweist, deren Produkt $\rho \cdot c \leq 0{,}6$ cal cm$^{-3}$ K$^{-1}$ ($\hat{=}$ $2{,}5 \cdot 10^6$ J m$^{-3}$ K$^{-1}$) ist.

6. Anordnung nach Anspruch 1, gekennzeichnet dadurch,
daß zur Erhöhung der Indikationsempfindlichkeit zusätzlich eine temperaturhochempfindliche, bei der Wellenlänge $\lambda_0$ der gefährdenden Strahlung mindestens nahezu absorptionsfreie Schicht in Nähe der absorbierenden Resonatorschicht vorhanden ist.

7. Anordnung nach Anspruch 6, gekennzeichnet dadurch,
daß die zusätzliche, temperaturhochempfindliche Schicht der absorbierenden Resonatorschicht unmittelbar vorgeordnet ist.

8. Anordnung nach Anspruch 6, gekennzeichnet dadurch,
daß die zusätzliche, temperaturhochempfindliche Schicht aus einem Polymer besteht.

9. Anordnung nach Anspruch 6, gekennzeichnet dadurch,
daß die zusätzliche, temperaturhochempfindliche Schicht im sichtbaren Spektralbereich eine die Wellenlänge $\lambda_0$ einschließende Absorptionsbande mit einer spektralen Halbwertsbreite kleiner als 200 nm aufweist.

10. Anordnung nach Anspruch 6, gekennzeichnet dadurch,
daß die Absorption der zusätzlichen, temperaturhochempfindlichen Schicht durch Einfärben hergestellt ist.

**Claims**

1. An arrangement for protection against electromagnetic radiation of at least one wavelength $\lambda_0$ incident at any angle, and consisting of a substrate transparent at least in the visible spectral range and a number of interference layers of different refractive indexes, characterised in that an absorbent resonator layer is additionally provided which, in conjunction with the interference layers of different refractive indexes, forms a sequence of optical layers which are selected and disposed on the principle of an optical thin-film resonance absorber system having at least one resonance wavelength $\lambda_r = \lambda_0$, the interference layers which are disposed after the absorbent resonator layer as considered in the direction of radiation being absorption-free at least in the spectral range between 450 nm and 750 nm and in that the absorbent resonator layer has a specific indication sensitivity $R_C$ so that if the incident radiation energy at wavelength $\lambda$ exceeds a threshold value $Q_{rad, min}$ detectable material changes occur, particularly as a result of photochemical or photothermal energy conversion effects.

2. An arrangement according to Claim 1, characterised in that the interference layers disposed after the absorbent resonator layer as considered in the direction of radiation consist of materials having alternately low and high or alternately high and low refractive indexes.

3. An arrangement according to Claim 2, characterised in that the optical thicknesses $n_S \cdot d_S$ of the interference layers disposed after the absorbent resonator layer as considered in the direction of radiation are dimensioned at $n_S \cdot d_S = (m+1) \lambda_0/4$, where $n_S$ denotes the refractive index of the individual layer, $d_S$ the geometric thickness of the individual layer and m is equal to zero or is a natural number
and
in that the interference layers disposed after the absorbent resonator layer as considered in the direction of radiation have, in a spectral interval containing the wavelength $\lambda_0$ and within the wavelength range $0.9 \cdot \lambda_0$ to $1.1 \cdot \lambda_0$, a degree of reflection with respect to air greater than 99% and, in the visible spectral range outside the wavelength range from $0.9 \lambda_0$ to $1.1 \cdot \lambda_0$, a degree of reflection with respect to air less than 50%, so that the partial sequence of optical layers disposed after the absorbent resonance layer are considered in the direction of radiation have, in their boundary surface with the resonance layer at wavelength $\lambda$, a pseudo-refractive index which is $\leq 0.1$ when the partial sequence starts with a layer having the small refractive index and which is $\geq 10$ when the partial sequence starts with a layer having the higher refractive index.

4. An arrangement according to Claim 1, characterised in that the material of the absorbent resonator layer has an absorption coefficient k greater than 1 at least at the wavelength $\lambda_0$.

5. An arrangement according to Claim 4, characterised in that the material of the absorbent resonator layer has a density $\rho$, and a specific heat c, the product of which is

$$\rho \cdot c \leq 0.6 \text{ cal cm}^{-3} \text{ K}^{-1} (\triangleq 2.5 \cdot 10^6 \text{ J m}^{-3} \text{K}^{-1}).$$

6. An arrangement according to Claim 1, characterised in that in order to increase the indication sensitivity, there is additionally provided in the vicinity of the absorbent resonator layer a layer which has high sensitivity to the temperature and which is at least almost absorption-free at the wavelength $\lambda_0$ of the hazardous radiation.

7. An arrangement according to Claim 6, characterised in that the additional layer having high temperature sensitivity is disposed immediately in front of the absorbent resonator layer.

8. An arrangement according to Claim 6, characterised in that the additional layer having high temperature sensitivity consists of a polymer.

9. An arrangement according to Claim 6, characterised in that the additional layer having high temperature sensitivity has in the visible spectral range an absorption band including the wavelength $\lambda_0$ and having a spectral half width less than 200 nm.

10. An arrangement according to Claim 6, characterised in that the absorption of the additional layer having high temperature sensitivity is produced by dyeing in.

**Revendications**

1. Système de protection contre un rayonnement électromagnétique d'au moins une longueur d'onde $\lambda_0$ arrivant sous des angles quelconques d'incidence, se composant d'au moins un substrat transparent dans la plage spectrale visible et de plusieurs couches d'interférence ayant des indices différents de réfraction, caractérisé

en ce qu'il existe en plus une couche absorbante de résonance qui forme avec les couches d'interférence ayant des indices de réfraction différents une succession de couches optiques qui sont sélectionnées et disposées selon le principe d'un système optique d'absorption de la résonance en couche mince ayant au moins une longueur d'onde de résonance $\lambda_r = \lambda_0$, les couches d'interférence disposées en aval de la couche absorbante de résonance, dans le sens du rayonnement, étant exemptes d'absorption au moins dans la plage spectrale comprise entre 450 nm et 750 nm et
en ce que la couche absorbante de résonance présente une sensibilité déterminée d'indication $R_c$ de manière qu'en cas de dépassement de l'énergie incidente de rayonnement, des modifications de matière décelables à la longueur d'onde $\lambda$ d'un seuil $Q_{rad.min}$ se produisent en particulier par des effets photochimiques ou photothermiques de conversion d'énergie.

2. Système selon la revendication 1, caractérisé
en ce que les couches d'interférence disposées en aval de la couche absorbante de résonance, dans le sens du rayonnement, consistent en des matières ayant des indices de réfraction en alternance bas et élevés ou en alternance élevés et bas.

3. Système selon la revendication 2, caractérisé

en ce que les épaisseurs optiques $n_S \cdot d_S$ des couches d'interférence disposées en aval de la couche absorbante de résonance dans le sens du rayonnement sont dimensionnées de manière que $n_S \cdot d_S = (m+1) \lambda_0/4$, $n_S$ étant l'indice de réfraction particulier de la couche individuelle, $d_S$, l'épaisseur géométrique particulière de la couche individuelle et m étant égal à zéro ou étant un entier naturel et
en ce que les couches d'interférence disposées en aval de la couche absorbante de résonance dans le sens du rayonnement ont un facteur de réflexion vis-à-vis de l'air qui est supérieur à 99% dans un intervalle spectral comprenant la longueur d'onde $\lambda_o$ à l'intérieur de la plage des longueurs d'onde de $0,9 \cdot \lambda_o$ jusqu'à $1,1 \cdot \lambda_o$ et un facteur de réflexion vis-à-vis de l'air qui est inférieur à 50% dans la plage spectrale visible qui est à l'extérieur de la plage de longueurs d'onde de $0,9 \lambda_o$ jusqu'à $1,1 \cdot \lambda_o$, de manière que la séquence partielle de couches optiques disposée derrière la couche absorbante de résonance dans le sens du rayonnement ait dans leur surface limite voisine de la couche de résonance, à la longueur d'onde $\lambda$, un pseudo-indice de réfraction qui est $\leq 0,1$ lorsque la séquence partielle débute par une couche à faible indice de réfraction et qui est $\geq 10$ lorsque la séquence partielle débute par une couche à grand indice de réfraction.

4. Système selon la revendication 1, caractérisé
en ce que la matière de la couche absorbante de résonance présente à une longueur d'onde $\lambda_o$ un coefficient d'absorption k qui est supérieur à 1.

5. Système selon la revendication 4, caractérisé
en ce que la matière de la couche absorbante de résonance a une densité $\rho$ et une chaleur spécifique c dont le produit $\rho \cdot c$ est $\leq 0,6$ cal cm$^{-3}$ K$^{-1}$ ($\hat{=} 2,5 \cdot 10^6$ J m$^{-3}$K$^{-1}$).

6. Système selon la revendication 1, caractérisé
en ce qu'il existe en plus, à proximité de la couche absorbante de résonance, une couche très sensible à la température, au moins approximativement exempte d'absorption à la longueur d'onde $\lambda_o$ du rayonnement dangereux afin d'élever la sensibilité d'indication.

7. Système selon la revendication 6, caractérisé
en ce que la couche supplémentaire, très sensible à la température, est disposée immédiatement devant la couche absorbante de résonance.

8. Système selon la revendication 6, caractérisé
en ce que la couche supplémentaire, très sensible à la température, est en un polymère.

9. Système selon la revendication 6, caractérisé
en ce que la couche supplémentaire, très sensible à la température, présente dans la plage spectrale visible une bande d'absorption incluant la longueur d'onde $\lambda_o$ et ayant une largeur spectrale de valeur moyenne inférieure à 200nm.

10. Système selon la revendication 6, caractérisé
en ce que l'absorption de la couche supplémentaire, très sensible à la température, est produite par coloration.

Fig. 1

Fig. 2

Fig.3

Fig.4

Fig.5